# EUROPEAN PATENT SPECIFICATION

(11) **EP 3 984 470 B1**
(45) Date of publication and mention of the grant of the patent: **01.07.2026**
(21) Application number: 21202360.0
(22) Date of filing: 13.10.2021
(51) Int. Cl.: A61B 17/00

(54) **SURGICAL IMPLANT FOR REPAIRING A DEFECT IN SPINAL DURA MATER**
CHIRURGISCHES IMPLANTAT ZUR REPARATUR EINES DEFEKTS IN DER HARTEN RÜCKENMARKSHAUT
IMPLANT CHIRURGICAL POUR LA RÉPARATION DE DÉFAUT DE LA DURE-MÈRE VERTÉBRALE

(30) Priority: 14.10.2020 US 202063198368 P; 12.10.2021 US 202117499844
(43) Date of publication of application: 20.04.2022
(73) Proprietor: Jenkins Neurospine LLC, New York, NY 10128 (US)
(72) Inventor: JENKINS, III, Arthur L., New York, NY 10128 (US)
(74) Representative: Swea IP Law AB

(56) References cited:
- US-A- 5 578 045
- US-A1- 2008 215 089
- US-A1- 2013 006 301
- US-B2- 8 500 776

## Description

### TECHNICAL FIELD

The present disclosure generally relates to a surgical implant. More specifically, the present disclosure relates to a surgical implant for repairing a defect in a spinal dura mater.

### BACKGROUND

The brain and spinal cord are bathed in fluid known as cerebrospinal fluid (CSF). This fluid is held inside layers of connective tissue called the meninges, which surround the brain and spinal cord. There are three meningeal layers: the pia mater; the arachnoid mater; and the dura mater. The dura mater, sometimes called the dura, is the outermost layer of the meninges. The dura is normally a tough connective tissue. The CSF is contained within the subarachnoid space, between the arachnoid mater and the pia mater layers. A spinal CSF leak happens when the spinal dura mater has a hole or tear, allowing CSF to leak out of this enclosed space. This results in intracranial hypotension, a low volume of CSF remaining around the brain and spinal cord. The loss of volume of CSF around the brain and spinal cord can result in a range of symptoms, with severe positional headache being the most common. Symptoms can be mild to very disabling. Often, a patient may have very limited ability to function while in the upright position. A wide range of neurologic signs and symptoms may occur, and rarely, dementia, stroke, coma, and even death have been reported.

During spine surgery, injury may occur due to instruments used to decompress nerves, misplaced implants or grafts, manipulation, and ischemia (compromised blood supply). The risk of having a spinal cord injury or a dural tear during spine surgery increases with age as the surgeon needs to cut through toughened spinal ligaments, and in the process, damage the dura. It may also occur in those with previous spine surgery or preexisting spinal cord compression. Surgery to the back of the spine has a higher risk of spinal cord injury or dural tears since it requires cutting through spinal ligaments, which may injure the dura or cord. Special precautions are taken before, during and after surgery to prevent a dural tear or spinal cord injury.

Dural tears are repaired using microsurgical techniques - using a microscope and a fine needle. Small dural tears are sutured or stapled close, while larger ones are reconstructed with a patch or graft. Fat or fibrin glue may be used as a sealant to reinforce the repair.

In some cases, surgical implants for repairing a defect in the dura mater are made of a collagen or collagen like materials. When the implants are applied, they conform to the complex surfaces of the brain or spinal cord. The patches are designed to interact with and often absorb the patient's blood and plasma exudate to promote healing in the tear of the dura. As the blood begins to clot adjacent to the dural patch, depending on the nature of the material the patch was made of, the dural patch is either slowly reabsorbed into the body and replaced by the patient's own dural tissue, or otherwise integrated in such a way that at least some dural tissue grows over the surface of the patch material to maintain a watertight seal. So too are vascular patches, which are either made of biological tissue, vein material, bovine epicardium, or other man-made materials that have the ability to let vascular endothelial layers cover the inner side, creating a smooth conduit that prevents clot formation within the repaired blood vessel.

Dural patches may be attached with sutures or simply be laid on the dural tear. A disadvantage of the methods currently used in clinical practice is that they only allow for the dural patches either to be laid on top of the tear, or sewn into the defect as a single layer, thus creating either minimal seal, or an opportunity for an uneven seal, or an initial seal that becomes leaky when the contents develop higher pressure including leaking at the suture line or suture holes because there are no additional layers to block leakage from the repair site. A further disadvantage of such implants is they may lead to tissue damage below the dura mater occurring as a result of the suturing process.

Thus, there exists a need in the art for an improved surgical implant to improve a defect in the dura mater.

### SUMMARY OF THE INVENTION

The needs set forth herein as well as further and other needs and advantages are addressed by the present teachings, which illustrate solutions and advantages described below.

An object of the present invention is to provide a spinal dura mater defect repairing surgical implant as defined in the independent claim 1. Preferred embodiments are defined in the dependent claims. This objective of the invention helps to keep internal (for example, neural) elements in place during the dural repair (by sealing them in with no egress of fluid which can carry nerves out the opening while the surgeon is trying valiantly to close the defect, making the repair that much harder still) or minimize active blood leakage from a vascular structure during the repair, which would reduce the need for occlusion of the parent vessel to allow the repair to occur. This will also lead to better long-term healing of the defect. Further, the inner portion of the dural patch may protect the nerves, or the vascular patch could reduce platelet aggregation and clot/thrombus formation, and then subsequently promote endothelization inside once the outer layer is sewn closed.

A further object of the present disclosure is a patch having a top and bottom layer. The bottom layer can change between two states, a relaxed state, and a tensioned state. In the relaxed state the top and bottom layer are substantially parallel to each other. In the tensioned state, the bottom layer will have been cinched such that its longitudinal perimeter is shorter, albeit the layer would appear thicker from a horizontal view. The cinched, modified shorter longitudinal perimeter of the bottom layer is designed to allow the bottom layer to fit inside a defect in a fluid-containing structure, while the top layer rests outside the outer layer of the structure, keeping it from falling deeper into the structure as well as being the site of the subsequent repair.

The bottom layer may be cinched using a suture threaded through the outside of the bottom layer, or other mechanisms as may be devised. In the case of the suture, once it is released from the inner layer and removed, the bottom layer will uncinch or expand back into the relaxed state and conform to the dura or vessel or tissue opposite the top layer. This results in a more effective seal to the defect than just resting a single layer on top of the dura or tissue or sewing a single layer into or over the defect. The suture may be threaded through the bottom layer in premade holes, premade indentures that make puncturing the inner layer of the patch easier or sewn through without any formal guidance.

The patch may be made of collagen which allows for an organic seal to the dura, vessel, or tissue. A collagen matrix of the patch allows blood and plasma to adhere to it, and form fibrin glue support the repair temporarily. The collagen matrix can then be resorbed harmlessly into the body as the body repairs and overlays the tissue with normal dura or endothelium and vascular tissue.

The third layer, a possible variation in the design, would provide a specific gap between the two layers, designed to match the thickness of the fluid-filled structure (artery, vein, or dura, for example) being repaired, so as not to cause bunching of the original tissue as the space between the outer and inner layers are pulled taut.

The present invention resides in a surgical implant for repairing a defect in a spinal dura mater as defined in claim 1. The implant includes a first layer having a top surface and a bottom surface. The first layer is flexible and planar. The first layer has an edge extending between the top surface of the first layer and the bottom surface of the first layer about a periphery of the first layer. The first layer has an inner portion remote from the periphery of the first layer. The first layer has an outer portion between the periphery of the first layer and the inner portion of the first layer. The surgical implant includes a second layer having a top surface and a bottom surface. The second layer is flexible and planar. The second layer has an edge extending between the top surface of the second layer and the bottom surface of the second layer about a periphery of the second layer. The second layer has an inner portion remote from the periphery of the second layer. The second layer has an outer portion between the periphery of the second layer and the inner portion of the second layer. The bottom surface of the first layer proximate to the inner portion thereof is connected to the top surface of the second layer proximate to the inner portion thereof so that the inner portion of the top layer is fixed relative to the inner portion of the second layer and the outer portion of the first layer is moveable relative to the outer portion of the second layer. Similar devices, not used for dura mater defect repair, are known from US5578045, US2008/215089 or US2103/006301.

According to the present invention, the first layer comprises a collagen-based dura graft material, and the second layer comprises a collagen-based dura graft material.

According to the present invention, the spinal dura mater has a defect area and an area of the connection between the bottom surface of the first layer and the top surface of the second layer is less than or equal to the defect area.

Further according to the present invention, the bottom surface of the first layer proximate to the outer portion thereof is configured to be positioned adjacent to an outer surface of the spinal dura mater proximate to the defect therein when the surgical implant is received in the defect. The top surface of the second layer proximate to the outer portion thereof is adjacent to an inner surface of the spinal dura mater proximate to the defect therein when the surgical implant is received in the defect.

Further according to the present invention, the edge of the second layer extends at least to the edge of the first layer when the surgical implant is configured to received in the defect in the spinal dura mater so that the outer portion of the second layer inhibits a damage to arachnoid mater below the dura mater resulting from a suturing of the outer portion of the first layer and the dura mater.

Further according to the present invention, the outer portion of the first layer of the implant is suitable to be fixed relative to the spinal dura mater with a suture between the outer portion of the first layer and the spinal dura mater proximate to the defect.

In yet a further embodiment of the present invention, the second layer is biasable between a retracted position and an extended position. In the retracted position the edge of the second layer about the periphery thereof is drawn together under the inner portion of the second layer thereby facilitating receipt of the second layer through the defect in the spinal dura mater. In the extended position the second layer extends in a plane that is generally parallel to the surface of the dura mater.

In yet a further embodiment of the present invention, a surgical thread is received in the outer portion of the second layer about the periphery thereof. When the second layer is in the retracted position, the surgical thread is tensioned to maintain the second layer in the retracted state. The second layer is biasable to the extended position when the tension is released from the surgical thread.

In yet a further embodiment of the present invention the surgical thread is dissolvable in the body after implantation thereof.

In yet a further embodiment of the present invention, the surgical implant comprises a third layer having a top surface and a bottom surface, the third layer being flexible and planar, the third layer being disposed between the first layer and the second layer, the third layer forming at least in part the connection between the first layer and the second layer.

Another aspect of the present disclosure not part of the present invention concerns a method for repairing a defect in a spinal dura mater of a patient. The method includes the step of applying a surgical implant to the defect in the spinal dura matter. The implant includes a first layer having a top surface and a bottom surface. The first layer is flexible and planar. The first layer has an edge extending between the top surface of the first layer and the bottom surface of the first layer about a periphery of the first layer. The first layer has an inner portion remote from the periphery of the first layer. The first layer has an outer portion between the periphery of the first layer and the inner portion of the first layer. The surgical implant includes a second layer having a top surface and a bottom surface. The second layer is flexible and planar. The second layer has an edge extending between the top surface of the second layer and the bottom surface of the second layer about a periphery of the second layer. The second layer has an inner portion remote from the periphery of the second layer. The second layer has an outer portion between the periphery of the second layer and the inner portion of the second layer. The bottom surface of the first layer proximate to the inner portion thereof is connected to the top surface of the second layer proximate to the inner portion thereof so that the inner portion of the top layer is fixed relative to the inner portion of the second layer and the outer portion of the first layer is moveable relative to the outer portion of the second layer.

In yet a further embodiment of disclosed method, the first layer comprises a collagen-based dura graft material, and the second layer comprises a collagen-based dura graft material.

In yet a further embodiment of disclosed method, the defect in the spinal dura mater has a defect area, and an area of the connection between the bottom surface of the first layer and the top surface of the second layer is less than or equal to the defect area.

In yet a further embodiment of disclosed method, the bottom surface of the first layer proximate to the outer portion thereof is adjacent to an outer surface of the spinal dura mater proximate to the defect therein when the implant is received in the defect. The top surface of the second layer proximate to the outer portion thereof is adjacent to an inner surface of the spinal dura mater proximate to the defect therein when the implant is received in the defect.

In yet a further embodiment of disclosed method, the method includes the step of suturing the outer portion of the first layer of the implant to the spinal dura mater proximate to the defect.

In yet a further embodiment of disclosed method, the edge of the second layer extends at least to the edge of the first layer when the surgical implant is received in the defect in the spinal dura mater so that the outer portion of the second layer inhibits a damage to an arachnoid mater below the dura mater resulting from a suturing of the outer portion of the first layer and the dura mater.

In yet a further embodiment of disclosed method, the second layer is biasable between a retracted position and an extended position. In the retracted position the edge of the second layer about the periphery thereof is drawn together under the inner portion of the second layer thereby facilitating receipt of the second layer through the defect in the spinal dura mater. In the extended position the second layer extends in a plane that is generally parallel to the surface of the dura mater.

In yet a further embodiment of disclosed method, the method includes the step of releasing a surgical thread received in the outer portion of the second layer about the periphery thereof. The releasing of the surgical thread causes the second layer to bias from the retracted position to the extended position.

In yet a further embodiment of disclosed method, the surgical thread is dissolvable in the body after implantation thereof.

In yet a further embodiment of disclosed method, the implant further comprises a third layer having a top surface and a bottom surface. The third layer is flexible and planar, the third layer is disposed between the first layer and the second layer. The third layer forms, at least in part, the connection between the first layer and the second layer.

These and other aspects of the present invention will become apparent in light of the drawings and detailed description provided below.

### BRIEF DESCRIPTION OF THE DRAWINGS

FIG. 1A illustrates a top view of a defect in a dura matter of a spinal cord.
FIB. 1B shows a cross sectional view through the plane 1B-1B of the spinal cord as indicated in FIG. 1A.
FIG. 2A is a side view of a surgical implant in accordance with one embodiment of the present invention.
FIG. 2B is a top view of the surgical implant shown in FIG. 2A.
FIG. 2C is a front view of the surgical implant shown in FIG. 2A.
FIG. 3A is a side view of a surgical implant in accordance with another embodiment of the present invention.
FIG. 3B is a top view of the surgical implant shown in FIG. 3A.
FIG. 3C is a front view of the surgical implant shown in FIG. 3A.
FIG. 4A is an isometric view of the surgical implant shown in FIG. 2A wherein the second layer is in the extended position.
FIG. 4B is an isometric view of the surgical implant shown in FIG. 2A wherein the second layer is in the retracted position.
FIG. 5A is a side view of the surgical implant shown in FIG. 2A wherein the second layer is in the extended position.
FIG. 5B is a side view of the surgical implant shown in FIG. 2A wherein the second layer is in the retracted position.
FIG. 6 is a view illustrating a method of installing the surgical implant in accordance with one embodiment of the present invention wherein the second layer is in the retracted position.
FIG. 7 is a view illustrating a method of installing the surgical implant in accordance with one embodiment of the present invention wherein the second layer is in the extended position and received in a defect in the dura mater.
FIG. 8A is a view of the surgical implant received in a defect in dura matter.
FIG. 8B shows a cross sectional view through the plane 8B-8B of the surgical implant received in a defect in dura matter.
FIG. 9A is a view of the surgical implant received in a defect in dura of FIG. 8A wherein the first layer of the implant is sutured to the dura mater.
FIG. 9B shows a cross sectional view through the plane 9B-9B of the surgical implant received in a defect in dura matter wherein the first layer of the implant is sutured to the dura mater.

### DETAILED DESCRIPTION

The present teachings are described more fully hereinafter with reference to the accompanying drawings. The following description is presented for illustrative purposes only and the present teachings should not be limited to these embodiments.

In compliance with the statute, the present teachings have been described in language more or less specific as to structural and methodical features. It is to be understood, however, that the present teachings are not limited to the specific features shown and described, since the systems and methods herein disclosed comprise preferred forms of putting the present teachings into effect.

For purposes of explanation and not limitation, specific details are set forth such as particular architectures, interfaces, techniques, etc. in order to provide a thorough understanding. In other instances, detailed descriptions of well-known devices, circuits, and methods are omitted so as not to obscure the description with unnecessary detail.

Generally, all terms used in the claims are to be interpreted according to their ordinary meaning in the technical field, unless explicitly defined otherwise herein. All references to a/an/the element, apparatus, component, means, step, etc. are to be interpreted openly as referring to at least one instance of the element, apparatus, component, means, step, etc., unless explicitly stated otherwise. The steps of any method disclosed herein do not have to be performed in the exact order disclosed, unless explicitly stated. The use of "first', 'second,' etc. for different features/components of the present disclosure are only intended to distinguish the features/components from other similar features/components and not to impart any order or hierarchy to the features/components.

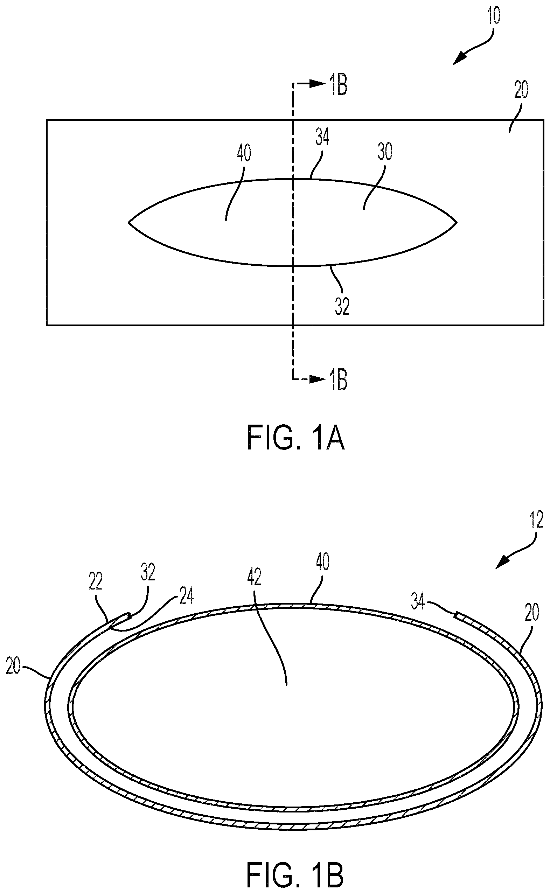

FIGS. 1A and 1B show a diagram representing a portion of a spinal cord 10 of a human. The spinal cord 10 is covered by dura mater 20. Dura mater is a thick membrane made of dense irregular connective tissue that surrounds the brain and spinal cord. It is the outermost of the three layers of membrane called the meninges that protect the central nervous system. The other two meningeal layers are the arachnoid mater and the pia mater. The dura mater has a defect therein 30. In FIG. 1A. the defect is a tear in the dura matter 20. The tear 30 may be caused naturally, or by a number of different medical interventions. The tear 30 has a first side 32 and a second side 34. The tear 30 extends along an axis from a first point to a second point. It will be understood to a person of ordinary skill in the art and familiar with this disclosure that the shape of the defect 30 may vary and that FIG. 1A and 1B are provided for illustrated purposes. The tear 30 in the dura mater 20 exposes the layers below the dura mater, namely the arachnoid mater and the pia mater. These sub-layers are illustrated by the reference number 42 and 40, which designates the outside surface thereof. The outside surface of the dura mater proximate to the tear is indicated by reference number 22. The inside surface of the dura mater proximate to the tear is indicated by reference number 24. It will be understood to a person of ordinary skill in the art that while the present invention is disclosed in the repair of a dural tear, the present invention is not limited in this regard. The implant in accordance with the present invention may also be used in the repair of defects in veins and arteries.

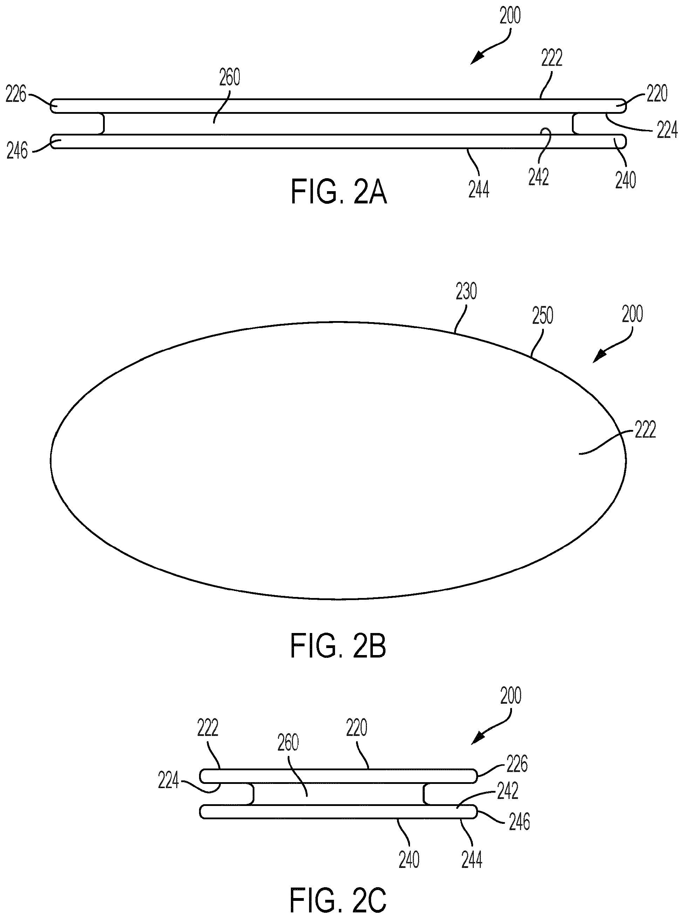

FIGS. 2A-2C illustrate a surgical implant 200 in accordance with one embodiment of the present invention. The surgical implant 200 has a first layer 220, a second layer 240, and a third layer 260 disposed between the first layer and the second layer. The first layer 220 has a perimeter 230 that extends beyond the third layer 260. The bottom layer 240 has a perimeter 250 that extends beyond the third layer 260.

The first layer 220 has a top surface 222 and a bottom surface 224. The first layer 220 is flexible. The first layer 220 extends in a plane. The first layer 220 has an edge 226 between the top surface 222 and the bottom surface 224. The edge 226 extends along a periphery 230 of the first layer 220. In reference to FIG. 2B, the periphery 230 of the first layer 220 defines an ellipse in the planar surface. It should be understood that the present invention is not limited in this regard. For example, , the first layer 220 may form a circular shape, a rectangular shape, or any other shape that is useful in addressing the defect in the tissue. In some embodiments of the present invention, the layers of the surgical implant are provided in a plurality of different sizes to allow the surgeon to select the appropriate size to use with a specific defect. In yet other embodiments of the present invention, the layers are provided in a stock size that can be customized for a specific defect by trimming the layers prior to use thereof.

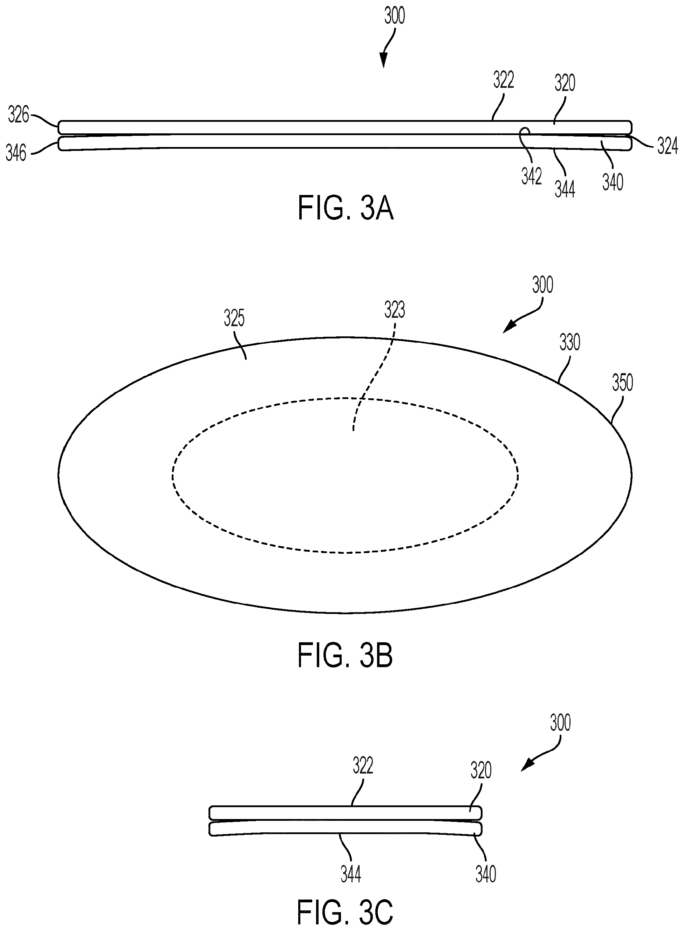

The first layer 220 has an inner portion remote from the periphery 230 of the first layer. The inner portion and area thereof may vary in different embodiments of the present invention. In reference to the embodiment shown in FIG. 3B, an inner portion of a first layer is illustrated by hidden lines. The first layer 220 has an outer portion between the periphery 230 of the first layer 220 and the inner portion of the first layer. In some embodiments, the outer portion defines an elliptical annulus in the plane of the first layer 220, wherein the outer extent of the annulus is the periphery 230. The inner area of the annulus is the inner portion of the first layer 220.

The second layer 240 has a top surface 242 and a bottom surface 244. The second layer 240 is flexible. The second layer 240 extends in a plane. The second layer 240 has an edge 246 between the top surface 242 and the bottom surface 244. The edge 246 extends along a periphery 250 of the second layer 240. In reference to FIG. 2B, the periphery 250 of the second layer 240 defines an ellipse in the planar surface. It should be understood that the present invention is not limited in this regard. For example, the second layer may form a circular shape, a rectangular shape, or any other shape that is useful in addressing the defect in the tissue. In some embodiments of the present invention, the layers of the surgical implant are provided in a plurality of different sizes to allow the surgeon to select the appropriate size to use with a specific defect. In yet other embodiments of the present invention, the layers are provided in a stock size that can be customized for a specific defect by trimming the layers prior to use thereof.

The second layer 240 has an inner portion remote from the periphery 250 of the second layer. The inner portion and area thereof may vary in different embodiments of the present invention. The second layer 240 has an outer portion between the periphery 250 of the second layer 240 and the inner portion of the second layer. In some embodiments, the outer portion defines an elliptical annulus in the plane of the second layer 220, wherein the outer extent of the annulus is the periphery 250. The inner area of the annulus is the inner portion of the second layer 220.

The bottom surface 224 of the first layer 220 proximate to the inner portion thereof is connected to the top surface 242 of the second layer 240 proximate to the inner portion thereof so that the inner portion of the first layer is fixed relative to the inner portion of the second layer and the outer portion of the first layer is moveable relative to the outer portion of the second layer. In the embodiment disclosed in FIGS. 2A-2C, connection between the first layer 220 and the second layer comprises a third layer 260. The third layer 260 is flexible and planar. In the embodiment disclosed in FIGS. 2A-2C, the area of the third layer 260 is commensurate in scope with area of the inner portion of the first layer 220 and the area of the inner portion of the second layer 240. The bottom surface 224 of the first layer 220 proximate to the inner portion thereof is connected to the top surface of third layer 260. The top surface 242 of the second layer 240 proximate to the inner portion thereof is connected to the bottom surface of the third layer 260.

In reference to FIG. 2A-2C, the outer portions of the first layer 220 and the second layer 240 extend past the extent of the connection between the first layer 220 and the second layer 240. In this manner the outer portion of the first layer 220 and the outer portion of the second layer 240 are moveable to each other due the flexible nature of the layer. Simultaneously, the inner portion of the first layer 220 and the inner portion of the second layer 240 are fixed relative to each other. The inventor has discovered that this configuration supports application of the surgical implant to the defect of the tissue.

Each layer may be made of a different material, each with different tissue or material properties (such as density, flexibility, or porosity), or each layer may be imbued or impregnated with different substances to perform the different tasks of healing innate to the different environments on the inside and outside of the target repaired tissue. In addition, the inner and outer layers may or may not be identical in size, shape, or thickness.

Numerous materials are known for forming the layers. A selected material should restore the continuity of the dura mater, prevents CSF leaks while minimizing infection, facilitates suturing, mimics the compliance of natural dura, and minimizes local tissue inflammation and encourages the infiltration of cells and vasculature to reconstruct native dura without inducing fibrosis or adhesions. Examples include, but are not limited to, autograft, allograft, xenograft, and non-biologic synthetic materials. Although dural substitutes such as autologous pericranium, bovine pericardium, cadaveric dura, autologous fascia lata, muscle have been widely used in cranial surgery, spine surgery generally has relied on using animal-derived or synthetic substitutes. Some examples include, but are not limited to, the following: Durasis, DuraGen or DuraMatrix, Durepair, DuraGuard, Alloderm, Preclude, and Neuro-Patch. Furthermore, Surgicel is sometimes used to augment some dural repairs.

The layer material comprises collagen-based xenografts, namely DuraGen, DuraMatrix, and Durepair. DuraGen (Integra Neuroscience, Plainsboro, NJ) and DuraMatrix (Stryker, Kalamazoo, MI) are synthetic substitutes consisting of type I collagen matrix made from bovine achilles tendon. DuraGen has 20% more conformability than DuraMatrix while DuraMatrix has a 50 times lower liquid permeability rate than DuraGen. DuraGen is perhaps now the most widely used dural substitute for spinal surgeries. DuraGen's porosity allows platelets to infiltrate the matrix and promote fibrin clot formation as well as fibroblasts to enter and lay down natural collagen fibers thus preventing CSF leakage and initiate the dural repair process. In humans, implanted DuraGen was completely resorbed within 1 year (often much earlier) and replaced by the host's collagen derived from infiltrating fibroblasts. It should be understood to a person of ordinary skill in the art that other biocompatible materials used. It should also be understood that additional materials or layers may be added to the surgical implant to increase the durability or stiffness of the product, as may be required. According to the present invention the first and second layers comprise a collaged-based graft material.

Examples of techniques for physically joining the layer 220, 240, 260 include pressing, heating, such as in an oven, with or without a vacuum, exposing the material to heating elements or heated air, or ultrasonically spot welding. The layers may be joined during processing of the collagen material. In some embodiments of the present invention, the layers may be joined using suture. In this manner, the layered implant may be prepared by a surgeon. In some embodiments of the present invention, the surgical implant is formed from a uniform layer of product, such as collagen, which is then subjected to an incision along the periphery, thereby defining the respective sections, namely the layers, of the surgical implant. In some embodiments of the present invention, the inner portions of the layers are connected with an adhesive or sealant. It should be understood that any known method of adhering the layers in accordance with the disclosure may be used and that the present invention is not limited in this regard.

In reference to FIGS. 3A-3C, an embodiment 300 of the present invention is shown in which the first layer 320 is directly connected the second layer 340. The first layer 320 has a top surface 322 and a bottom surface 324. The first layer 320 is flexible. In first layer 320 extends in a plane. The first layer 320 has an edge 326 between the top surface 322 and the bottom surface 324. The edge 326 extends along a periphery 330 of the first layer. In reference to FIG. 3C, the periphery 330 of the first layer 320 defines an ellipse in the planar surface. It should be understood that the present invention is not limited in this regard. For example, the, the first layer 320 may form a circular shape, a rectangular shape, or any other shape that is useful in addressing the defect in the tissue. In some embodiments of the present invention, the layers of the surgical implant are provided in a plurality of different sizes to allow the surgeon to select the appropriate size to use with a specific defect. In yet other embodiments of the present invention, the layers are provided in a stock size that can be customized for a specific defect by trimming the layers prior to use thereof.

The first layer 320 has an inner portion 323 remote from the periphery 330 of the first layer. The inner portion and area thereof may vary in different embodiments of the present invention. The first layer 320 has an outer portion 325 between the periphery 330 of the first layer 320 and the inner portion 323 of the first layer. In some embodiments, the outer portion 325 defines an elliptical annulus in the plane of the first layer 320, wherein the outer extent of the annulus is the periphery 330. The inner area of the annulus is the inner portion 323 of the first layer 320.

The second layer 340 has a top surface 342 and a bottom surface 344. The second layer 340 is flexible. The second layer 340 extends in a plane. The second layer 340 has an edge 346 between the top surface 342 and the bottom surface 344. The edge 346 extends along a periphery 350 of the second layer 340. In reference to FIG. 2B, the periphery 350 of the second layer 340 defines an ellipse in the planar surface. It should be understood that the present invention is not limited in this regard. For example, the, the second layer may form a circular shape, a rectangular shape, or any other shape that is useful in addressing the defect in the tissue. In some embodiments of the present invention, the layers of the surgical implant are provided in a plurality of different sizes to allow the surgeon to select the appropriate size to use with a specific defect. In yet other embodiments of the present invention, the layers are provided in a stock size that can be customized for a specific defect by trimming the layers prior to use thereof.

The second layer 340 has an inner portion remote from the periphery 350 of the second layer. The inner portion and area thereof may vary in different embodiments of the present invention. The second layer 340 has an outer portion between the periphery 350 of the second layer 340 and the inner portion of the second layer. In some embodiments, the outer portion defines an elliptical annulus in the plane of the second layer 320, wherein the outer extent of the annulus is the periphery 350. The inner area of the annulus is the inner portion of the second layer 320.

The bottom surface 324 of the first layer 320 proximate to the inner portion thereof is connected to the top surface 342 of the second layer 340 proximate to the inner portion thereof so that the inner portion of the first layer is fixed relative to the inner portion of the second layer and the outer portion of the first layer is moveable relative to the outer portion of the second layer. In the embodiment disclosed in FIGS. 3A-3C, the first layer 320 is directly adjacent to the second layer.

In reference to FIG. 3A-3C, the outer portions of the first layer 320 and the second layer 340 extend past the extent of the connection between the first layer 320 and the second layer 340. In this manner the outer portion of the first layer 320 and the outer portion of the second layer 340 are moveable to each other due the flexible nature of the layers. Simultaneously, the inner portion of the first layer 320 and the inner portion of the second layer 340 are fixed relative to each other. The inventor has discovered that this configuration supports application of the surgical implant to the defect of the tissue.

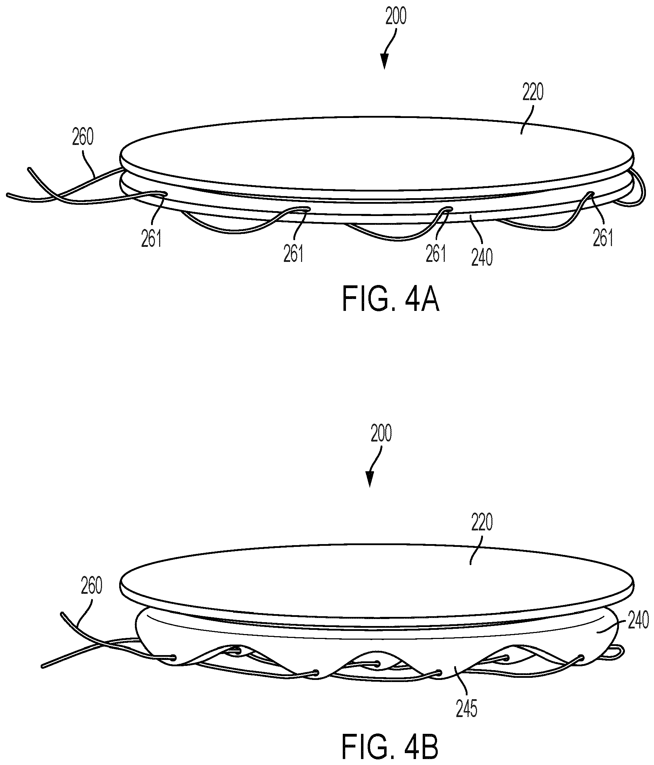

FIG. 4A illustrates an embodiment of the surgical implant 200 in the relaxed state. In the relaxed state, also referred to as the deployed state, the first layer 220 and the second layer 240 are substantially parallel to each other. In one embodiment of the invention, the second layer 240 may return to the relaxed state when no external force is applied to the second layer 240.

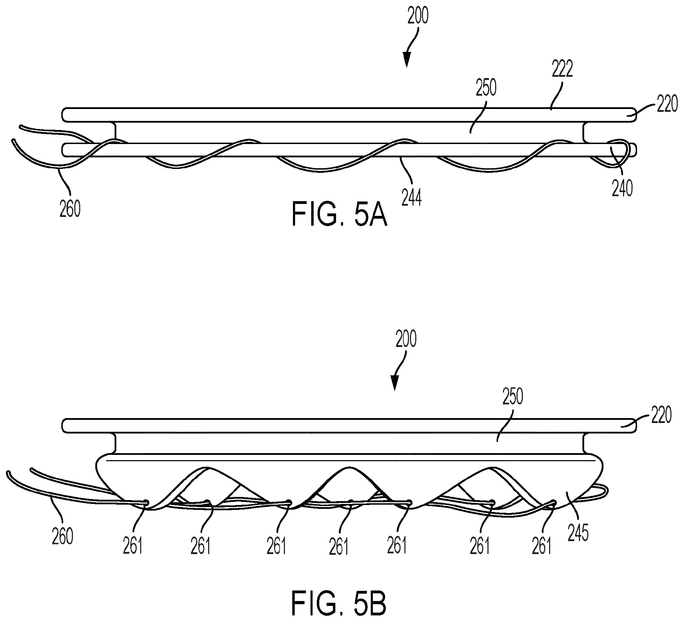

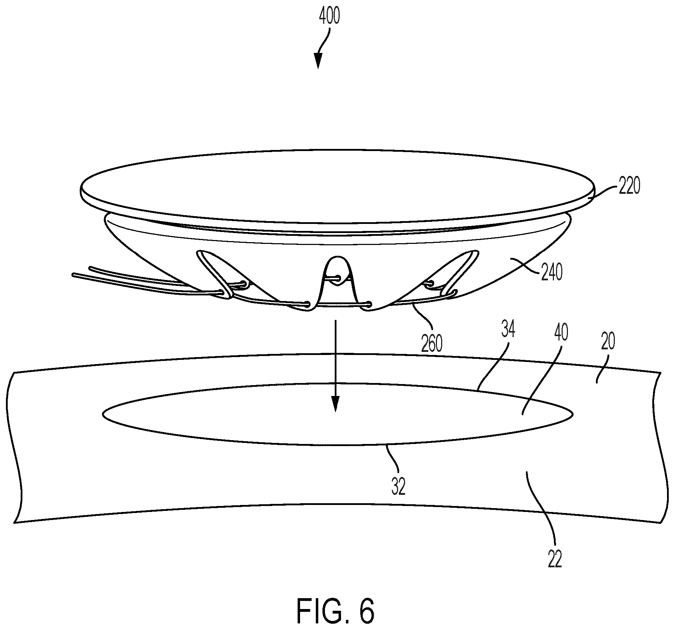

In one embodiment of the present invention, the second layer 220 includes a plurality of holes 261 in the outer portion thereof. The holes 261 extend along the periphery of the second layer 240. A surgical thread 260 is received through the holes 261 so that the length of surgical thread 260 extends along the periphery of the surgical implant. In some embodiments of the present invention, the surgical thread is dissolvable. In this manner, the second layer 240 is biasable between a retracted position and an extended position. In the retracted position, shown partially in FIG. 4B, 5B, and shown more fully in FIG. 6, the edge of the second layer 240 about the periphery thereof is drawn together under the inner portion of the second layer 240 thereby facilitating receipt of the second layer 240 through the defect. In the embodiment disclosed, the retracted position may be achieved by tensioning the surgical thread. The extended position is illustrated in FIGS. 4A, 5A, 7, 8A-B, and 9A-9B. In the extended position the second layer 240 extends in a plane that is generally parallel to the defect. The term generally parallel is used to indicate that the surface of the dura mater naturally may, for example, have a convex arcuate surface in a plane perpendicular to the spinal column. The second layer 240 is generally parallel to the defect when it aligns with this surface.

Holes 161 may be through the entire layer 3 or only a starter hole to guide a suture or other device through the holes. In some objects of the invention the holes may not be in place until after a suture, needle, or other object has passed through the second layer. The holes 161 may be scattered along the perimeter of the second layer 240. In one object of the invention, each hole may be the same length away from the edge of second layer 240. In another object, the holes may be scattered more at random through the second layer. In one object of the invention the holes 161 are used to cinch the second layer 240 as described above.

Figure 4B, 5B, and 5 depicts a suture 160 threaded through the holes 161 of the second layer such that the second layer 240 is in a tensioned state. As the suture 160 is tightened the entire second layer is cinched centrally. As shown in figures, the effective perimeter of the second layer has been shortened based on how taut the suture 160 has been pulled.

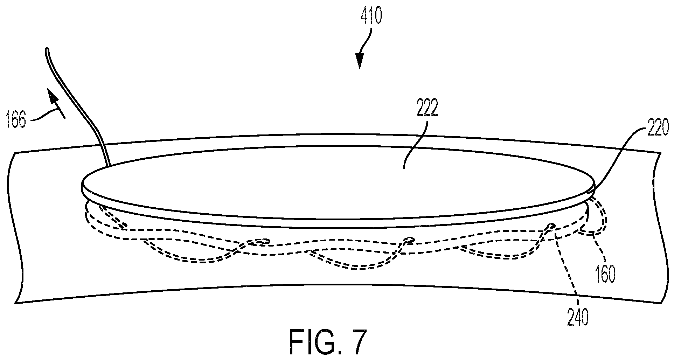

In the embodiment disclosed, the area of the connection between the top layer and the bottom layer, is less than the area of the defect in the tissue. By retracting the second layer 240 in this manner, the present invention enables the second layer, having a great area in the defect, to be received through the aperture defined by the defect. After the second layer 240 is received below the defect 30, the second layer is biased to the extended state by releasing tension on the surgical thread. In reference to FIG. 6, the method of inserting the implant into the defect is illustrated, wherein the second layer is in the retracted configuration. Although the present embodiment is illustrated with a surgical thread 160 to bias the position of the second layer 240, the present invention is not limited in this regard. For example, the second layer may be folded over to facilitate receipt thereof into the defect. The second layer 220 may subsequently be deployed using surgical equipment and force imparted thereby on the implant and adjacent tissue. In reference to FIG. 7, the implant is shown biased toward the configuration wherein the outer portion of the second layer 220 is extended. In some embodiments of the present invention, as the surgical thread is pulled from the second layer 240, the natural conforming nature of the second layer will allow the second layer to expand back to its original shape against a bottom wall of the defect. Depending on the shape of the structure being repaired, the second layer may enter into the complete relaxed state, or a state similar to the relaxed state against the underside of the tissue being repaired.

As would be understood by one skilled in the art, the amount the perimeter that is able to be cinched will depend on the structural integrity of the material is made of as well as the distance the holes are from the edge of bottom layer, as well as the tension applied.

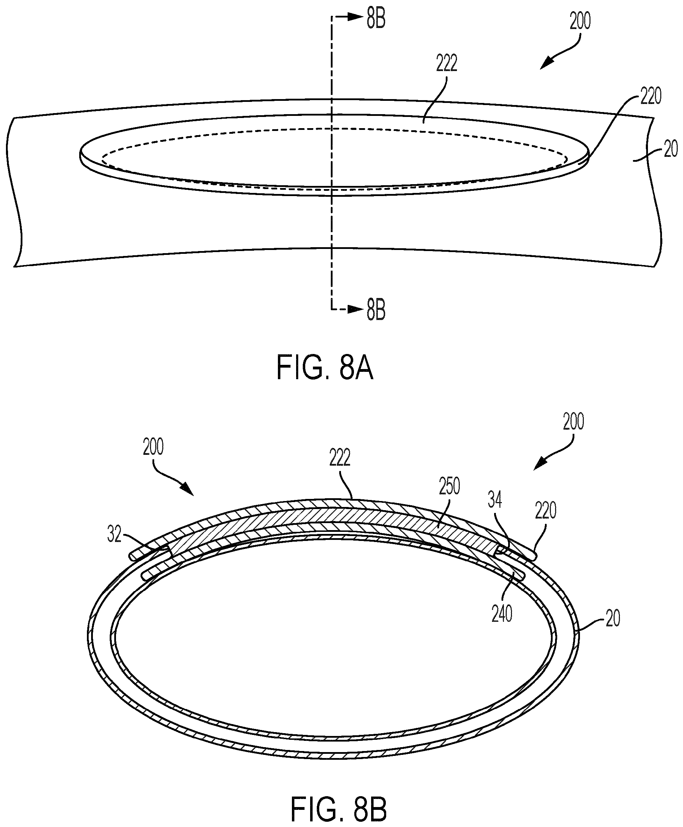

In reference to FIGS. 8A and 8B, the surgical implant is shown deployed in a defect 30 in dura mater. The area of the first layer 220 is greater than the area of the defect 30. The area of the second layer 240 is greater than the area of defect. In this manner the portion of the dura mater proximate to opposing sides 32, 34 of the defect 30 is received between the first layer 220 and the second layer 240 proximate to the outer portion of the first layer and the second layer. This configuration is illustrated, for example, in reference to the cross-sectional view shown in FIG. 8B. In this manner, the inventor has discovered that the surgical implant in accordance with the present invention inhibits the leakage of CSF from the defect dressed with the surgical implant 220. The bottom surface 224 of the first layer proximate to the outer portion thereof is adjacent to an outer surface of the spinal dura mater 20 proximate to the defect 30 therein when the surgical implant 200 is received in the defect. The top surface of the second layer 240 proximate to the outer portion thereof is adjacent to an inner surface of the spinal dura mater 20 proximate to the defect therein when the surgical implant is received in the defect.

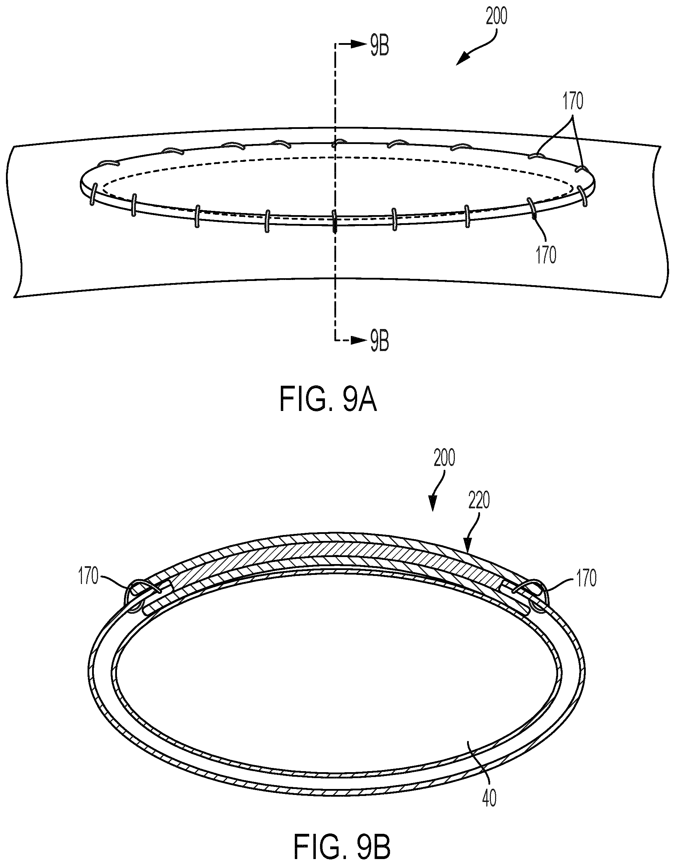

In reference to FIG. 9A and 9B a further embodiment of the present invention is illustrated wherein the surgical implant is connected to the dura mater via a suture 170. It should be understood that although a suture step is shown in this connection with the embodiment disclosed in FIG. 9A and 9B the present invention is not limited in this regard. For example, in some embodiments of the present invention, the surgical implant is retained in the defect through pressure and friction. In the embodiment disclosed in FIGS. 9A-9B. The outer portion of the first layer 220 of the implant 220 is fixed relative to the spinal dura mater with a suture between the outer portion of the first layer 220 and the spinal dura mater proximate to the defect 30. The inventor has discovered that the second layer 240 below the dura matter as illustrated in FIG. 9B, services to inhibit damage caused to the sub- dura layers from the suturing and associated equipment by inhibiting a penetration thereof. This results in improved recovery and CSF leak inhibition. It should be understood that the area of the second layer relative to the first layer may vary. In this embodiment, the edge of the second layer extends at least to the edge of the first layer when the surgical implant is received in the defect in the spinal dura mater so that the outer portion of the second layer inhibits a damage to arachnoid mater below the dura mater resulting from a suturing of the outer portion of the first layer and the dura mater.

The suture keeps the surgical implant 200 in place so the dura or vessel can heal over the patch. In one object of the invention, the implant may be made of collagen such that blood and plasma can adhere to the collagen matrix. In some embodiments of the present invention, in about 6-8 weeks the collagen matrix will be resorbed and integrated to the tissue. This forms a successful graft repairing the defect.

The sutures are looped through the first layer 220 and through the dura. In one embodiment of the invention a sticky, glue or sealant may be applied, or some texture that promotes otherwise adherence to the dura or hearing. In such an embodiment, the bottom layer 3 may cause resistance to the removal of the dural patch, as it has expanded to a perimeter beyond the perimeter of the wound.

The present invention may use any suture material known in the art. Four examples that are used by spine surgeons to repair dura are Nurolon^{®} (Ethicon, Inc., Sommerville, NJ), Prolene^{®} (Ethicon, Inc., Sommerville, NJ), Gore-Tex^{®} (W. L. Gore & Associates, Inc., Flagstaff, Arizona) and Silk. Nurolon is a non-absorbable, braided suture composed of the long-chain aliphatic polymer of Nylon 6 or Nylon 6,6. It is noted to have 81% tensile strength at 1 year, 72% at 2 years, and 66% at 11 years and elicits minimal acute inflammatory reaction. Prolene is a non-absorbable monofilament suture composed of an isotactic crystalline stereoisomer of polypropylene, a synthetic linear polyolefin. This material does not adhere to tissues, is biologically inert, and elicits minimal tissue reaction and maintains tensile strength for up to 2 years. Gore-Tex suture is a microporous, non-absorbable monofilament made of expanded polytetrafluoroethylene (ePTFE) and its unique structure allows the attachment of needles that approximate the diameter of the thread and is therefore thought to fill the entire needle hole, thus reducing CSF leak at the suture site. Finally, silk suture is made of raw silk spun by silkworms and, although classified as non-absorbable, silk suture becomes absorbed by proteolysis and is often undetectable in the wound site by 2 years. Although several examples are provided here, the present disclosure is not limited in this regard. The described examples may also be used to bias the second layer of the surgical implant.

The present disclosure describes aspects of the invention with reference to the exemplary embodiments illustrated in the drawings; however, aspects of the invention are not limited to the exemplary embodiments illustrated in the drawings. It will be apparent to those of ordinary skill in the art that aspects of the invention include many more embodiments. Accordingly, aspects of the invention are not to be restricted in light of the exemplary embodiments illustrated in the drawings. It will also be apparent to those of ordinary skill in the art that variations and modifications can be made without departing from the scope of the present claims. For example, in some instances, one or more features disclosed in connection with one embodiment can be used alone or in combination with one or more features of one or more other embodiments.

## Claims

1. A spinal dura matter (20) defect repairing surgical implant (200), the implant comprising:
a first layer (220,320) having a top surface (222,322) and a bottom surface (224,324), the first layer being flexible and planar, the first layer having an edge extending between the top surface of the first layer and the bottom surface of the first layer about a periphery(230,330) of the first layer, the first layer having an inner portion remote from the periphery of the first layer, the first layer having an outer portion between the periphery (230,330) of the first layer and the inner portion of the first layer;
a second layer (240,340) having a top surface (242,342) and a bottom surface (244,344), the second layer being flexible and planar, the second layer having an edge extending between the top surface of the second layer and the bottom surface of the second layer about a periphery (250,350) of the second layer, the second layer having an inner portion remote from the periphery of the second layer, the second layer having an outer portion between the periphery (250,350) of the second layer and the inner portion of the second layer;
wherein the first layer (220,320) comprises a collagen-based graft material, and
wherein the second layer (240,340) comprises a collagen-based graft material, wherein the bottom surface (224,324) of the first layer (220,320) proximate to the inner portion thereof is connected to the top surface (242,342) of the second layer (240,340) proximate to the inner portion thereof so that the inner portion of the top layer (242,342) is fixed relative to the inner portion of the second layer (240,340) and the outer portion of the first layer (220,320) is moveable relative to the outer portion of the second layer (240,340),
wherein an area of the connection between the bottom surface (224,324) of the first layer (220,320) and the top surface (242,342) of the second layer(240, 340) is less than or equal to an area of the defect (30),
wherein the bottom surface (224,324) of the first layer (220,320) proximate to the outer portion thereof is configured to be positioned adjacent to an outer surface of the spinal dura mater (20) proximate to the defect (30) therein when the surgical implant (200) is received in the defect,
wherein the top surface (242,342) of the second layer (240,340) proximate to the outer portion thereof is configured to be positioned adjacent to an inner surface of the spinal dura mater (20) proximate to the defect (30) therein when the surgical implant is received in the defect, wherein the edge (246,346) of the second layer (240,340) extends at least to the edge (226,326) of the first layer (220,320) as determined in a horizontal plane for each respective layer,
wherein the outer portion of the first layer (220,320) of the implant (200) is configured to be fixed relative to the spinal dura mater (20) with a suture (170) between the outer portion of the first layer and the spinal dura mater (20) proximate to the defect (30).

2. The surgical implant (200) of claim 1,
wherein the second layer (240,340) is biasable between a retracted position and an extended position,
wherein in the retracted position the edge (246,346) of the second layer about the periphery (250,350) thereof is drawn together under the inner portion of the second layer thereby facilitating receipt of the second layer through the defect (30),
wherein in the extended position the second layer is suitable to extend in a plane that is generally parallel to the defect.

3. The surgical implant (200) of claim 2, further comprising:
a surgical thread (160) received in the outer portion of the second layer about the periphery (250,350) thereof;
wherein when the second layer (240,340) is in the retracted position, the surgical thread is tensioned to maintain the second layer in the retracted state;
wherein the second layer is biasable to the extended position when the tension is released from the surgical thread.

4. The surgical implant (200) of claim 1, further comprising:
a third layer (260) having a top surface and a bottom surface, the third layer being flexible and planar, the third layer being disposed between the first layer (220) and the second layer (240), the third layer forming at least in part the connection between the first layer and the second layer.

## Patentansprüche

1. Chirurgisches Implantat (200) zur Reparatur eines Defekts der spinalen Dura mater (20), wobei das Implantat Folgendes umfasst:
eine erste Schicht (220,320), die eine obere Oberfläche (222,322) und eine untere Oberfläche (224,324) aufweist, wobei die erste Schicht flexibel und planar ist, wobei die erste Schicht eine Kante aufweist, die sich zwischen der oberen Oberfläche der ersten Schicht und der unteren Oberfläche der ersten Schicht um einen Umfang (230,330) der ersten Schicht erstreckt, wobei die erste Schicht einen inneren Abschnitt aufweist, der von dem Umfang der ersten Schicht entfernt ist, wobei die erste Schicht einen äußeren Abschnitt zwischen dem Umfang (230,330) der ersten Schicht und dem inneren Abschnitt der ersten Schicht aufweist;
eine zweite Schicht (240,340), die eine obere Oberfläche (242,342) und eine untere Oberfläche (244,344) aufweist, wobei die zweite Schicht flexibel und planar ist, wobei die zweite Schicht eine Kante aufweist, die sich zwischen der oberen Oberfläche der zweiten Schicht und der unteren Oberfläche der zweiten Schicht um einen Umfang (250,350) der zweiten Schicht erstreckt, wobei die zweite Schicht einen inneren Abschnitt aufweist, der von dem Umfang der zweiten Schicht entfernt ist, wobei die zweite Schicht einen äußeren Abschnitt zwischen dem Umfang (250,350) der zweiten Schicht und dem inneren Abschnitt der zweiten Schicht aufweist;
wobei die erste Schicht (220,320) ein kollagenbasiertes Transplantatmaterial umfasst und
wobei die zweite Schicht (240,340) ein kollagenbasiertes Transplantatmaterial umfasst,
wobei die untere Oberfläche (224,324) der ersten Schicht (220,320) nahe deren innerem Abschnitt mit der oberen Oberfläche (242,342) der zweiten Schicht (240,340) nahe deren innerem Abschnitt verbunden ist, so dass der innere Abschnitt der oberen Schicht (242,342) relativ zu dem inneren Abschnitt der zweiten Schicht (240,340) fixiert ist und der äußere Abschnitt der ersten Schicht (220,320) relativ zu dem äußeren Abschnitt der zweiten Schicht (240,340) beweglich ist,
wobei eine Fläche der Verbindung zwischen der unteren Oberfläche (224,324) der ersten Schicht (220,320) und der oberen Oberfläche (242,342) der zweiten Schicht (240, 340) kleiner oder gleich einer Fläche des Defekts (30) ist,
wobei die untere Oberfläche (224,324) der ersten Schicht (220,320) nahe deren äußerem Abschnitt konfiguriert ist, um angrenzend an eine äußere Oberfläche der spinalen Dura mater (20) nahe dem Defekt (30) darin positioniert zu werden, wenn das chirurgische Implantat (200) in dem Defekt aufgenommen ist,
wobei die obere Oberfläche (242,342) der zweiten Schicht (240,340) nahe deren äußerem Abschnitt konfiguriert ist, um angrenzend an eine innere Oberfläche der spinalen Dura mater (20) nahe dem Defekt (30) darin positioniert zu werden, wenn das chirurgische Implantat in dem Defekt aufgenommen ist, wobei sich die Kante (246,346) der zweiten Schicht (240,340) mindestens bis zu der Kante (226,326) der ersten Schicht (220,320) erstreckt, wie in einer horizontalen Ebene für jede jeweilige Schicht bestimmt,
wobei der äußere Abschnitt der ersten Schicht (220,320) des Implantats (200) konfiguriert ist, um relativ zu der spinalen Dura mater (20) mit einer Naht (170) zwischen dem äußeren Abschnitt der ersten Schicht und der spinalen Dura mater (20) nahe dem Defekt (30) fixiert zu werden.

2. Chirurgisches Implantat (200) nach Anspruch 1,
wobei die zweite Schicht (240,340) zwischen einer zurückgezogenen Position und einer ausgefahrenen Position vorspannbar ist,
wobei in der zurückgezogenen Position die Kante (246, 346) der zweiten Schicht um deren Umfang (250, 350) unter dem inneren Abschnitt der zweiten Schicht zusammengezogen wird, wodurch die Aufnahme der zweiten Schicht durch den Defekt (30) erleichtert wird,
wobei die zweite Schicht in der ausgefahrenen Position geeignet ist, sich in einer Ebene zu erstrecken, die im Allgemeinen parallel zu dem Defekt ist.

3. Chirurgisches Implantat (200) nach Anspruch 2, ferner umfassend:
einen chirurgischen Faden (160), der in dem äußeren Abschnitt der zweiten Schicht um deren Umfang (250,350) aufgenommen ist;
wobei, wenn sich die zweite Schicht (240,340) in der zurückgezogenen Position befindet, der chirurgische Faden gespannt ist, um die zweite Schicht in dem zurückgezogenen Zustand zu halten;
wobei die zweite Schicht in die ausgefahrene Position vorspannbar ist, wenn die Spannung von dem chirurgischen Faden gelöst wird.

4. Chirurgisches Implantat (200) nach Anspruch 1, ferner umfassend:
eine dritte Schicht (260), die eine obere Oberfläche und eine untere Oberfläche aufweist, wobei die dritte Schicht flexibel und planar ist, wobei die dritte Schicht zwischen der ersten Schicht (220) und der zweiten Schicht (240) angeordnet ist, wobei die dritte Schicht zumindest teilweise die Verbindung zwischen der ersten Schicht und der zweiten Schicht bildet.

## Revendications

1. Implant chirurgical (200) de réparation de défauts de la dure-mère spinale (20), l'implant comprenant :
une première couche (220, 320) ayant une surface supérieure (222, 322) et une surface inférieure (224, 324), la première couche étant souple et plane, la première couche ayant un bord s'étendant entre la surface supérieure de la première couche et la surface inférieure de la première couche autour d'une périphérie (230, 330) de la première couche, la première couche ayant une partie interne éloignée de la périphérie de la première couche, la première couche ayant une partie externe entre la périphérie (230, 330) de la première couche et la partie interne de la première couche ;
une deuxième couche (240, 340) ayant une surface supérieure (242, 342) et une surface inférieure (244, 344), la deuxième couche étant souple et plane, la deuxième couche ayant un bord s'étendant entre la surface supérieure de la deuxième couche et la surface inférieure de la deuxième couche autour d'une périphérie (250, 350) de la deuxième couche, la deuxième couche ayant une partie interne éloignée de la périphérie de la deuxième couche, la deuxième couche ayant une partie externe entre la périphérie (250, 350) de la deuxième couche et la partie interne de la deuxième couche ;
dans lequel la première couche (220, 320) comprend un matériau de greffe à base de collagène, et
dans lequel la deuxième couche (240, 340) comprend un matériau de greffe à base de collagène,
dans lequel la surface inférieure (224, 324) de la première couche (220, 320) à proximité de la partie interne de celle-ci est reliée à la surface supérieure (242, 342) de la deuxième couche (240, 340) à proximité de la partie interne de celle-ci de sorte que la partie interne de la couche supérieure (242, 342) est fixe par rapport à la partie interne de la deuxième couche (240, 340) et la partie externe de la première couche (220, 320) est mobile par rapport à la partie externe de la deuxième couche (240, 340),
dans lequel une zone de la liaison entre la surface inférieure (224, 324) de la première couche (220, 320) et la surface supérieure (242, 342) de la deuxième couche (240, 340) est inférieure ou égale à une zone du défaut (30),
dans lequel la surface inférieure (224, 324) de la première couche (220, 320) à proximité de la partie externe de celle-ci est conçue pour être positionnée adjacente à une surface externe de la dure-mère spinale (20) à proximité du défaut (30) dans celle-ci lorsque l'implant chirurgical (200) est reçu dans le défaut,
dans lequel la surface supérieure (242, 342) de la deuxième couche (240, 340) à proximité de la partie externe de celle-ci est conçue pour être positionnée adjacente à une surface interne de la dure-mère spinale (20) à proximité du défaut (30) lorsque l'implant chirurgical est reçu dans le défaut, dans lequel le bord (246, 346) de la deuxième couche (240, 340) s'étend au moins jusqu'au bord (226, 326) de la première couche (220, 320) telle que déterminée dans un plan horizontal pour chaque couche respective,
dans lequel la partie externe de la première couche (220, 320) de l'implant (200) est conçue pour être fixée par rapport à la dure-mère spinale (20) avec une suture (170) entre la partie externe de la première couche et la dure-mère spinale (20) à proximité du défaut (30).

2. Implant chirurgical (200) selon la revendication 1,
dans lequel la deuxième couche (240, 340) peut être sollicitée entre une position rétractée et une position étendue,
dans lequel dans la position rétractée le bord (246, 346) de la deuxième couche autour de la périphérie (250, 350) de celle-ci est rapproché sous la partie interne de la deuxième couche facilitant ainsi la réception de la deuxième couche à travers le défaut (30),
dans lequel dans la position étendue, la deuxième couche est adaptée pour s'étendre dans un plan qui est généralement parallèle au défaut.

3. Implant chirurgical (200) selon la revendication 2, comprenant en outre :
un fil chirurgical (160) reçu dans la partie externe de la deuxième couche autour de la périphérie (250, 350) de celle-ci ;
dans lequel lorsque la deuxième couche (240, 340) est dans la position rétractée, le fil chirurgical est tendu pour maintenir la deuxième couche dans l'état rétracté ;
dans lequel la deuxième couche peut être sollicitée en position étendue lorsque la tension est relâchée du fil chirurgical.

4. Implant chirurgical (200) selon la revendication 1, comprenant en outre :
une troisième couche (260) ayant une surface supérieure et une surface inférieure, la troisième couche étant souple et plane, la troisième couche étant disposée entre la première couche (220) et la deuxième couche (240), la troisième couche formant au moins en partie la liaison entre la première couche et la deuxième couche.
